# EUROPEAN PATENT APPLICATION

(11) **EP 2 270 002 A1**
(43) Date of publication of application: **05.01.2011**
(21) Application number: 09251594.9
(22) Date of filing: 18.06.2009
(51) Int. Cl.: C07D 405/04, C07D 409/04, C07D 295/033, A61K 31/517, A61P 29/00

(54) **Quinazoline derivatives as histamine H4-receptor inhibitors for use in the treatment of inflammatory disorders**

(71) Applicant: Vereniging voor Christelijk Hoger Onderwijs, Wetenschappelijk Onderzoek en Patiëntenzorg, 1081 HV Amsterdam (NL)
(72) Inventor: The designation of the inventor has not yet been filed
(74) Representative: Perry, Robert Edward

(57) **Abstract**

Compounds that modulate the histamine H₄ receptor, and which may be useful for treating or preventing disorders and conditions mediated by the histamine H₄ receptor, e.g. inflammation, are of formula (I) 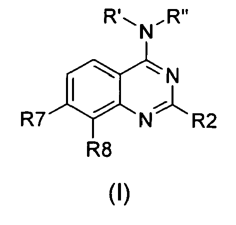 Wherein
R2 is H or NRxRy, where Rx and Ry are each independently selected from H, C₁₋₆ alkyl and (CH₂)₁₋₃C₃₋₇cycloalkyl; or Rx and Ry, together with the nitrogen atom to which they are bound form a 4, 5 or 6 membered heterocyclic group;
NR'R" is one of the following moieties, wherein R' and R" are taken together or separately as defined by each of one said moieties: 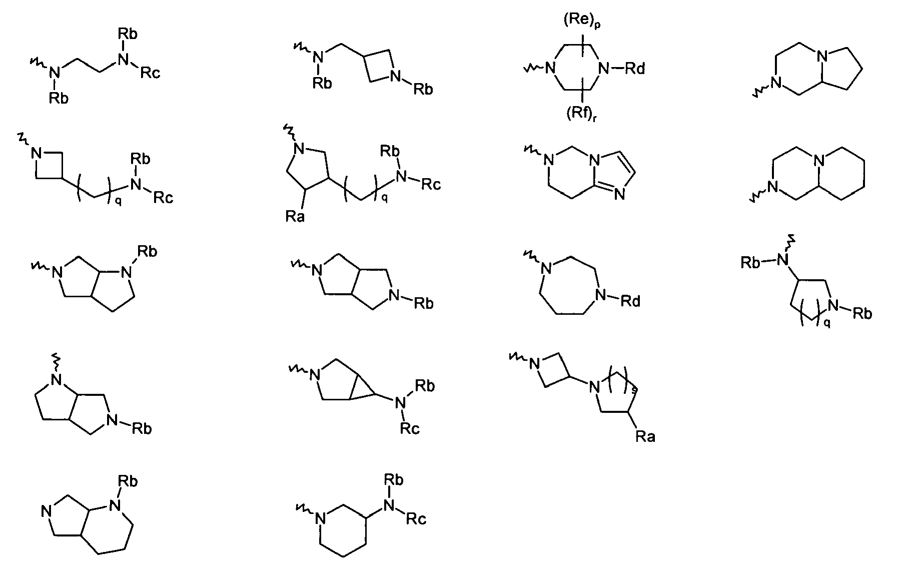 q is 0 or 1;
p is 0 or 1;
r is 0 or 1;
s is 0 or 1;
Ra is H or OH or F;
Rb and Rc are each independently H or C₁₋₃ alkyl;
Rd is H or OH or NH₂;
Re and Rf are each methyl, or Re and Rf taken together form a methylene or ethylene bridge; and
R₇ and R₈ are independently selected form H, F, Cl, Br, I, C₁₋₄ alkyl, C₂₋₅ alkenyl, C₂₋₅ alkynyl, C₁₋₄ alkoxy, C₁₋₄ alkylthio, (CH₂)₀₋₃C₃₋₇cycloalkyl, O-C₃₋₆ cycloalkyl, phenyl, benzyl, O-phenyl, NH-phenyl, S-phenyl, O-C₁₋₄ alkyl-phenyl, C₁₋₄ alkyl-phenyl, CF₃, O-CF₃, S-CF₃, hydroxy, nitro, cyano, O-C₁₋₄ alkyl-N(CH₃)₂, and NRmRn, wherein Rm and Rn are independently selected from H, C₁₋₄ alkyl, phenyl, benzyl and phenethyl, and wherein any phenyl, alkyl or cycloalkyl moiety is optionally substituted with 1 to 3 substituents selected from C₁₋₃ alkyl, halogen, hydroxy, amino and C₁₋₃ alkoxy;
or pharmaceutically acceptable salt, ester, or solvate thereof.

## Description

### Field of the Invention

The present invention relates to compounds based on the quinazoline scaffold, and their therapeutic use. More particularly, it relates to compounds, pharmaceutical compositions containing them, and methods of using them for the modulation of the histamine H₄ receptor and for the treatment of disease states, disorders and conditions mediated by histamine H₄ receptor activity.

### Background of the Invention

The histamine H₄ receptor (H₄R) is the most recently identified receptor for histamine (Fung-Leung, W.-P., et al., Curr. Opin. Invest. Drugs 2004, 5(11), 1174-1183; de Esch, I. J. P., et al., Trends Pharmacol. Sci. 2005, 26(9), 462-469). The receptor is found in the bone marrow and spleen and is expressed on eosinophils, basophils, mast cells (Liu, C., et al., Mol. Pharmacol. 2001, 59(3), 420-426; Liu, C., et al., J. Pharmacology and Experimental Therapeutics 2001, 299, 121-130; Morse, K. L., et al., J. Pharmacol. Exp. Ther. 2001, 296(3), 1058-1066; Hofstra, C. L., et al., J. Pharmacol. Exp. Ther. 2003, 305(3), 1212-1221; Lippert, U., et al., J. Invest. Dermatol. 2004, 123(1), 116-123; Voehringer, D., et al., Immunity 2004, 20(3), 267-277), CD8⁺ T cells (Gantner, F., et al., J. Pharmacol. Exp. Ther. 2002, 303(1), 300-307), dendritic cells, and human synovial cells from rheumatoid arthritis patients (Ikawa, Y., et al., Biol. Pharm. Bull. 2005, 28(10), 2016-2018). However, expression in neutrophils and monocytes is less well defined (Ling, P., et al., Br. J. Pharmacol. 2004, 142(1), 161-171). Receptor expression is at least in part controlled by various inflammatory stimuli (Coge, F., et al., Biochem. Biophys. Res. Commun. 2001, 284(2), 301-309; Morse, *et al.,* 2001), thus supporting that H₄ receptor activation influences inflammatory responses. Because of its preferential expression on immunocompetent cells, the H₄ receptor is closely related with the regulatory functions of histamine during the immune response.

A biological activity of histamine in the context of immunology and autoimmune diseases is closely related with the allergic response and its deleterious effects, such as inflammation. Events that elicit the inflammatory response include physical stimulation (including trauma), chemical stimulation, infection, and invasion by a foreign body. The inflammatory response is characterised by pain, increased temperature, redness, swelling, reduced function, or a combination of these.

Mast cell degranulation (exocytosis) releases histamine and leads to an inflammatory response that may be initially characterised by a histamine-modulated wheal and flare reaction. A wide variety of immunological stimuli (e.g., allergens or antibodies) and non-immunological (e.g., chemical) stimuli may cause the activation, recruitment, and de-granulation of mast cells. Mast cell activation initiates allergic inflammatory responses, which in turn cause the recruitment of other effector cells that further contribute to the inflammatory response. It has been shown that histamine induces chemotaxis of mouse mast cells (Hofstra, *et al.,* 2003). Chemotaxis does not occur using mast cells derived from H₄ receptor knockout mice. Furthermore, the response is blocked by an H₄-specific antagonist, but not by H₁, H₂ or H₃ receptor antagonists (Hofstra, *et al*., 2003; Thurmond, R. L., et al., J. Pharmacol. Exp. Ther. 2004, 309(1), 404-413). The in vivo migration of mast cells to histamine has also been investigated and shown to be H₄-receptor dependent (Thurmond, *et al.,* 2004). The migration of mast cells may play a role in allergic rhinitis and allergy where increases in mast cell number are found (Kirby, J. G., et al., Am. Rev. Respir. Dis. 1987, 136(2), 379-383; Crimi, E., et al., Am. Rev. Respir. Dis. 1991, 144(6), 1282-1286; Amin, K., et al., Am. J. Resp. Crit. Care Med. 2000, 162(6), 2295-2301; Gauvreau, G. M., et al., Am. J. Resp. Crit. Care Med. 2000, 161(5), 1473-1478; Kassel, O., et al., Clin. Exp. Allergy 2001, 31(9), 1432-1440). In addition, it is known that in response to allergens there is a redistribution of mast cells to the epithelial lining of the nasal mucosa (Fokkens, W. J., et al., Clin. Exp. Allergy 1992, 22(7), 701-710; Slater, A., et al., J. Laryngol. Otol. 1996, 110, 929-933). These results show that the chemotactic response of mast cells is mediated by histamine H₄ receptors.

It has been shown that eosinophils can chemotax towards histamine (O'Reilly, M., et al., J. Recept. Signal Transduction 2002, 22(1-4), 431-448; Buckland, K. F., et al., Br. J. Pharmacol. 2003, 140(6), 1117-1127; Ling *et al*., 2004). Using H₄ ligands, it has been shown that histamine-induced chemotaxis of eosinophils is mediated through the H₄ receptor (Buckland, *et al.,* 2003; Ling *et al*., 2004). Cell surface expression of adhesion molecules CD11 b/CD18 (LFA-1) and CD54 (ICAM-1) on eosinophils increases after histamine treatment (Ling, *et al*., 2004). This increase is blocked by H₄-receptor antagonists but not by H₁, H₂, or H₃ receptor antagonists.

The H₄R also plays a role in dendritic cells and T cells. In human monocyte-derived dendritic cells, H₄R stimulation suppresses IL-12p70 production and drives histamine-mediated chemotaxis (Gutzmer, R., et al., J. Immunol. 2005, 174(9), 5224-5232). A role for the H₄ receptor in CD8⁺ T cells has also been reported. Gantner, *et al.,* (2002) showed that both H₄ and H₂ receptors control histamine-induced IL-16 release from human CD8⁺ T cells. IL-16 is found in the bronchoalveolar fluid of allergen- or histamine-challenged asthmatics (Mashikian, V. M., et al., J. Allergy Clin. Immunol. 1998, 101 (6, Part 1), 786-792; Krug, N., et al., Am. J. Resp. Crit. Care Med. 2000, 162(1), 105-111) and is considered important in CD4⁺ cell migration. The activity of the receptor in these cell types indicates an important role in adaptive immune responses such as those active in autoimmune diseases.

In vivo H₄ receptor antagonists were able to block neutrophillia in zymosan-induced peritonitis or pleurisy models (Takeshita, K., et al., J. Pharmacol. Exp. Ther. 2003, 307(3), 1072-1078; Thurmond, *et al.,* 2004). In addition, H₄ receptor antagonists have activity in a widely used and well-characterised model of colitis (Varga, C., et al., Eur. J. Pharmacol. 2005, 522(1-3), 130-138). These results support the conclusion that H₄ receptor antagonists have the capacity to be anti-inflammatory in vivo.

Another physiological role of histamine is as a mediator of itch and H₁ receptor antagonists are not completely effective in the clinic. Recently, the H₄ receptor has also been implicated in histamine-induced scratching in mice (Bell, J. K., et al., Br. J. Pharmacol. 2004, 142(2), 374-380). The effects of histamine could be blocked by H₄ antagonists. These results support the hypothesis that the H₄ receptor is involved in histamine-induced itch and that H₄ receptor antagonists will therefore have positive effects in treating pruritis.

Modulation of H₄ receptors controls the release of inflammatory mediators and inhibits leukocyte recruitment, thus providing the ability to prevent and/or treat H₄-mediated diseases and conditions, including the deleterious effects of allergic responses such as inflammation. Compounds according to the present invention have H₄ receptor modulating properties. Compounds according to the present invention have leukocyte recruitment inhibiting properties. Compounds according to the present invention have anti-inflammatory properties.

Small-molecule histamine H₄ receptor modulators according to this invention control the release of inflammatory mediators and inhibit leukocyte recruitment, and may be useful in treating inflammation of various etiologies, including the following conditions and diseases: inflammatory disorders, allergic disorders, dermatological disorders, autoimmune disease, lymphatic disorders, pruritis, and immunodeficiency disorders. Diseases, disorders and medical conditions that are mediated by histamine H₄ receptor activity include those referred to herein.

WO2008/009078 describes quinazoline derivatives for treating viral infections. Further prior art in this general area includes WO2005/105761, WO98/02434, WO2006/039718 WO2008/009078 and W02009/047255.

### Summary of the Invention

In one aspect, the invention relates to compounds of formula (I) 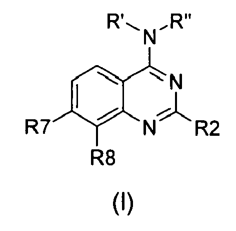 Wherein
R2 is H or NRxRy, where Rx and Ry are each independently selected from H, C₁₋₆ alkyl and (CH₂)₁₋₃C₃₋₇cycloalkyl; or Rx and Ry, together with the nitrogen atom to which they are bound form a 4, 5 or 6 membered heterocyclic group;
NR'R" is one of the following moieties, wherein R' and R" are taken together or separately as defined by each of one said moieties: 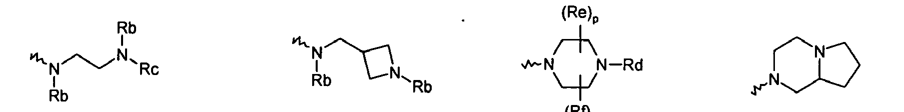 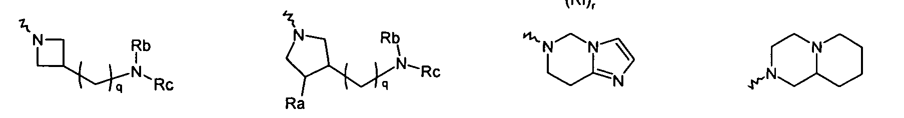 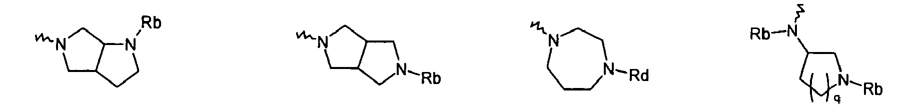 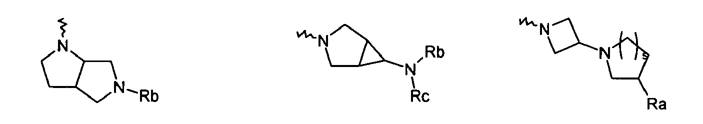 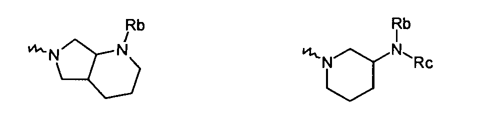 (The waved arrow in the figure above represents the molecular bond with which the said moieties are attached to the quinazoline 4-position).
q is 0 or 1;
p is 0 or 1;
r is 0 or 1;
s is 0 or 1 or 2;
Ra is H or OH or F;
Rb and Rc are each independently H or C₁₋₃ alkyl;
Rd is H or OH or NH₂;
Re and Rf are each methyl, or Re and Rf taken together form a methylene or ethylene bridge; and
R₇ or R₈ are independently selected from H, F, Cl, Br, I, C₁₋₆ alkyl, C₂₋₅ alkenyl, C₂₋₅ alkynyl, C₁₋₄ alkoxy, C₁₋₄ alkylthio, (CH₂)₀₋₃C₃₋₇cycloalkyl, O-C₃₋₆ cycloalkyl, aryl, heteroaryl O-phenyl, NH-phenyl, S-phenyl, O-C₁₋₄ alkyl-phenyl, C₁₋₄ alkyl-phenyl, CF₃, O-CF₃, S-CF₃, SO₂-C₃₋₇cycloalkyl, SO₂-C₁₋₇alkyl, SO₂-aryl, hydroxy, nitro, cyano, O-C₁₋₄ alkyl-N(CH₃)₂, and NRmRn, wherein Rm and Rn are independently selected from H, C₁₋₄ alkyl, phenyl, benzyl and phenethyl, and wherein any phenyl, alkyl or cycloalkyl moiety is optionally substituted with 1 to 3 substituents selected from C₁₋₃ alkyl, halogen, hydroxy, amino,or C₁₋₃ alkoxy.
R₇ or R₈ can also be independently selected from an aryl or heteroaryl group, optionally substituted with on or more of the following substituents: F, Cl, Br, I, C₁₋₄ alkyl, C₂₋₅ alkenyl, C₂₋₅ alkynyl, C₁₋₄ alkoxy, C₁₋₄ alkylthio, (CH₂)₀₋₃C₃₋₇cycloalkyl, O-C₃₋₆ cycloalkyl, phenyl, benzyl, O-phenyl, NH-phenyl, S-phenyl, O-C₁₋₄ alkyl-phenyl, C₁₋₄ alkyl-phenyl, CF₃, O-CF₃, S-CF₃, hydroxy, nitro, cyano or O-C₁₋₄ alkyl-N(CH₃)₂.

In some embodiments R₇ and R₈ can be taken together to form a 5-7 membered aliphatic ring, optionally substituted with 1 to 3 substituents selected from C₁₋₃ alkyl, trifluoromethyl, halogen, hydroxy or C₁₋₃ alkoxy;
or a pharmaceutically acceptable salt, ester, or solvate thereof.

According to a further aspect of the invention, compounds of formula (I) can be used to treat a subject suffering from or diagnosed with a disease, disorder or medical condition mediated by histamine H₄ receptor activity. For this purpose, they may be administered to a subject, e.g. as a pharmaceutical composition, in a therapeutically effective amount.

### Description of the Invention

Compounds of the invention may be chiral. This invention includes such compounds in any enantiomeric or diastereomeric form, including racemates. Compounds of the invention may also exist in different tautomeric forms, and all are included.

Preferably R2 is NH₂.

Preferably NR'R" is 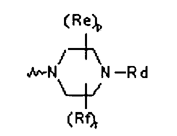 wherein preferably Rd is methyl and potentially Re and Rf form an ethylene bridge.

The term "alkyl" as used herein includes straight-chain and branched hydrocarbon groups.

The term "alkenyl" as used herein includes straight-chain and branched hydrocarbon groups as above with at least one carbon-carbon double bond (sp²).

The term "alkynyl" as used herein includes straight-chain and branched hydrocarbon groups as above with at least one carbon-carbon triple bond (sp). Hydrocarbons having a mixture of double bonds and triple bonds are grouped as alkynyls herein.

The term "alkoxy" as used herein includes straight-chain and branched alkyl groups with a terminal oxygen linking the alkyl group to the rest of the molecule.

The term "aryl" as used herein includes any functional group or substituent comprising an aromatic ring. In particular the aryl may be selected from moieties comprising a phenyl, naphtyl or biphenyl. The aryl may comprise one or more heteroatoms, in which case the aryl may be referred to as "heteroaryl". Preferred examples of heteroaryl groups include pyridine, furane, thiophene, pyrrole, imidazole, thiazole, oxazole, and triazole.

The term "cycloalkyl" as used herein includes saturated or partially saturated, monocyclic, fused polycyclic, or spiro polycyclic carbocycle having from 3 to 7 ring atoms per carbocycle.

The term "heterocyclic" refers to a monocyclic, or fused, bridged, or spiro polycyclic ring structure that is saturated or partially saturated and has from 4 to 8 ring atoms per ring structure selected from carbon atoms and up to three heteroatoms selected from nitrogen, oxygen and sulfur. The ring structure may optionally contain up to two oxo groups in carbon or sulfur ring members.

It is understood that substitutions and combinations of substitutions recited herein refer to substitutions that are consistent with the valency of the member being substituted.

The "pharmaceutically acceptable salt, ester or solvate thereof' refers to those salts, ester forms and solvates of the compounds of the present invention that would be apparent to the pharmaceutical chemist, i.e. those that are non-toxic and that would favourably affect the pharmacological properties of said compounds of the present invention. Those compounds having favourable pharmacological properties would be apparent to the pharmaceutical chemist, i.e. those that are non-toxic and that possess such pharmacological properties to provide sufficient palatability, absorption, distribution, metabolism and excretion. Other factors, more practical in nature, that are important in the selection are cost of raw materials, ease of crystallisation, yield, stability, hygroscopicity, and flowability of the resulting bulk drug.

Representative acids that may be used in the preparation of pharmaceutically acceptable salts include but are not limited to the following: acetic acid, 2,2-dichlorolactic acid, acylated amino acids, adipic acid, alginic acid, ascorbic acid, L-aspartic acid, benzenesulphonic acid, benzoic acid, 4-acetamidobenzoic acid, (+)-camphoric acid, camphorsulphonic acid, (+)-(1S)-camphor-10-sulphonic acid, capric acid, caproic acid, caprylic acid, cinnamic acid, citric acid, cyclamic acid, dodecylsulphuric acid, ethane-1,2-disulphonic acid, ethanesulphonic acid, 2-hydroxy-ethanesulphonic acid, formic acid, fumaric acid, galactaric acid, gentisic acid, glucoheptonic acid, D-gluconic acid, D-glucuronic acid, L-glutamic acid, α-oxo-glutaric acid, glycolic acid, hipuric acid, hydrobromic acid, hydrochloric acid, (+)-L-lactid acid, (±)-DL-lactic acid, lactobionic acid, maleic acid, (-)-L-malic acid, malonic acid, (±)-DL-mandelic acid, methanesulphonic acid, naphthalene-2-sulphonic acid naphthalene-1,5-disulphonic acid, 1-hydroxy-2-naphthoic acid, nicotinic acid, nitric acid, oleic acid, orotic acid, oxalic acid, palmitic acid, pamoic acid, phosphoric acid, L-pyroglutamic acid, salicylic acid, 4-amino-salicylic acid, sebacic acid, stearic acid, succinic acid, sulphuric acid, tannic acid, (+)-L-tartaric acid, thiocyanic acid, p-toluenesulphonic acid and undecylenic acid.

Representative bases that may be used in the preparation of pharmaceutically acceptable salts include the following: ammonia, L-arginine, benethamine, benzathine, calcium hydroxide, choline, deanol, diethanolamine, diethylamine, 2-(diethylamino)-ethanol, ethanolamine, ethylenediamine, N-methyl-glucamine, hydrabamine, 1H-imidazole, L-lysine, magnesium hydroxide, 4-(2-hydroxyethyl)-morpholine, piperazine, potassium hydroxide, 1-(2-hydroxyethyl)-pyrrolidine, secondary amine, sodium hydroxide, triethanolamine, tromethamine and zinc hydroxide.

Examples of suitable esters include C₁₋₇ alkyl, C₅₋₇ cycloalkyl, phenyl, substituted phenyl, and phenyl-C₁₋₆ alkyl esters. Preferred esters include methyl esters.

Any formula given herein is also intended to represent unlabeled forms as well as isotopically labeled forms of the compounds. Isotopically labeled compounds have structures depicted by the formulas given herein except that one or more atoms are replaced by an atom having a selected atomic mass or mass number. Examples of isotopes that can be incorporated into compounds of the invention include isotopes of hydrogen, carbon, nitrogen, oxygen, phosphorous, fluorine, chlorine, and iodine, such as ²H, ³H, ¹¹C, ¹³C, ¹⁴C, ¹⁵N, ¹⁸O, ¹⁷O, ³¹P, ³²P, ³⁵S, ¹⁸F, ³⁶Cl, ¹²⁵I, respectively. Such isotopically labelled compounds are useful in metabolic studies (preferably with ¹⁴C), reaction kinetic studies (with, for example ²H or ³H), detection or imaging techniques [such as positron emission tomography (PET) or single-photon emission computed tomography (SPECT)] including drug or substrate tissue distribution assays, or in radioactive treatment of patients. In particular, an ¹⁸F or ¹¹C labeled compound may be particularly preferred for PET or SPECT studies. Further, substitution with heavier isotopes such as deuterium (i.e., ²H) may afford certain therapeutic advantages resulting from greater metabolic stability, for example increased in vivo half-life or reduced dosage requirements. Isotopically labeled compounds of this invention can generally be prepared by substituting a readily available isotopically labeled reagent for a non-isotopically labeled reagent.

The present invention includes prodrugs of the compounds of the invention. In general, such prodrugs will be functional derivatives of the compounds that are readily convertible *in vivo* into the bio-active compound. Thus, in the uses of the compounds for methods of treatment of the present invention, the term "administering" shall encompass the treatment of the various disorders described with the compound specifically disclosed or with a compound that may not be specifically disclosed, but that converts to the specified compound *in vivo* after administration to the patient. Analogously, the term "compound", when applied to compounds of this invention, shall encompass any specific compound according to the present invention or any compound (or prodrug) that converts to the specifically disclosed compound *in vivo* after administration, even if such prodrug is not explicitly disclosed herein.

Examples of prodrugs include compounds having an amino acid residue, or a polypeptide chain of two or more (e.g., two, three or four) amino acid residues, covalently joined through an amide or ester bond to a free amino or hydroxyl group of a compound of formula (I). Examples of amino acid residues include the twenty naturally occurring amino acids, commonly designated by three letter symbols, as well as 4-hydroxyproline, hydroxylysine, demosine, isodemosine, 3-methylhistidine, norvalin, beta-alanine, gamma-aminobutyric acid, citrulline homocysteine, homoserine, ornithine and methionine sulfone.

The present invention also relates to pharmaceutically active metabolites of compounds of formula (I), and uses of such metabolites in the methods of the invention. A "pharmaceutically active metabolite" means a pharmacologically active product of metabolism in the body of a compound of formula (I) or salt thereof. Prodrugs and active metabolites of a compound may be determined using routine techniques known or available in the art. See, e.g., Bertolini, et al., J. Med. Chem. 1997, 40, 2011-2016; Shan, et al., J. Pharm. Sci. 1997, 86 (7), 765-767; Bagshawe, Drug Dev. Res. 1995, 34, 220-230; Bodor, Adv. Drug Res. 1984, 13, 224-331; Bundgaard, Design of Prodrugs (Elsevier Press, 1985); and Larsen, Design and Application of Prodrugs, Drug Design and Development (Krogsgaard-Larsen, et al., eds., Harwood Academic Publishers, 1991).

Tables 1 and 2 illustrate compounds of the invention.

**Table 1**

| | | | | | |
|---|---|---|---|---|---|
| 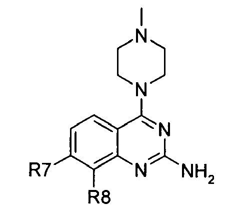 | | | | | |

| **No** | R₇ | Kᵢ | **No** | R₈ | Kᵢ |
|---|---|---|---|---|---|
| **1** | CH₃ | 24 | **10** | CH₃ | 91 |
| **2** | Br | 17 | **11** | Br | 111 |
| **3** | 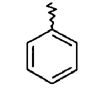 | 19 | **12** | 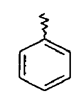 | 32 |
| **4** | 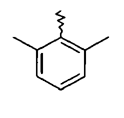 | 90 | **13** | 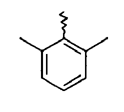 | 221 |
| **5** | 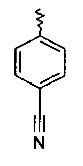 | 29 | **14** | 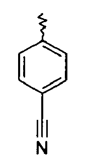 | 2333 |
| **6** | 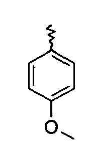 | 55 | **15** | 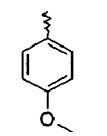 | 945 |
| **7** | 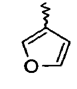 | 6 | **16** | 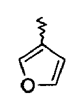 | 109 |
| **8** | 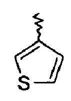 | 4 | | | |
| **9** | 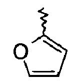 | 5 | | | |

Compounds of the present invention are useful as histamine H₄ receptor modulators in the methods of the invention. Such methods for modulating histamine H₄ receptor activity comprise exposing histamine H₄ receptor to an effective amount of at least one chemical entity selected from compounds of formula (I). Embodiments of this invention inhibit histamine H₄ receptor activity.

In some embodiments, the histamine H₄ receptor is in a subject with a disease, disorder, or medical condition mediated through modulation of the histamine H₄ receptor, such as those described herein. Symptoms or disease states are intended to be included within the scope of "medical conditions, disorders, or diseases."

Accordingly, the invention relates to methods of using the active agents described herein to treat subjects diagnosed with or suffering from a disease, disorder, or condition mediated through histamine H₄ receptor activity, such as inflammation. Active agents according to the invention may therefore be used as an anti-inflammatory agents.

In some embodiments, an active agent of the present invention is administered to treat inflammation. Inflammation may be associated with various diseases, disorders, or conditions, such as inflammatory disorders, allergic disorders, dermatological disorders, autoimmune disease, lymphatic disorders, and immunodeficiency disorders and cancer, including the more specific conditions and diseases given below. Regarding the onset and evolution of inflammation, inflammatory diseases or inflammation-mediated diseases or conditions include, but are not limited to, acute inflammation, allergic inflammation, and chronic inflammation.

Illustrative types of inflammation treatable with a histamine H₄ receptor-modulating agent according to the invention include inflammation due to any one of a plurality of conditions such as allergy, asthma, dry eye, chronic obstructed pulmonary disease (COPD), atherosclerosis, rheumatoid arthritis, multiple sclerosis, inflammatory bowel diseases (including colitis, Crohn's disease, and ulcerative colitis), psoriasis, pruritis, itchy skin, atopic dermatitis, urticaria (hives), ocular inflammation (e.g., post-surgical ocular inflammation), conjunctivitis, dry eye, nasal polyps, allergic rhinitis, nasal itch, scleroderma, autoimmune thyroid diseases, immune-mediated (also known as type 1) diabetes mellitus and lupus, which are characterised by excessive or prolonged inflammation at some stage of the disease. Other autoimmune diseases that lead to inflammation include Myasthenia gravis, autoimmune neuropathies, such as Guillain-Barré, autoimmune uveitis, autoimmune hemolytic anemia, pernicious anemia, autoimmune thrombocytopenia, temporal arteritis, anti-phospholipid syndrome, vasculitides, such as Wegener's granulomatosis, Behcet's disease, dermatitis herpetiformis, pemphigus vulgaris, vitiligio, primary biliary cirrhosis, autoimmune hepatitis, autoimmune oophoritis and orchitis, autoimmune disease of the adrenal gland, polymyositis, dermatomyositis, spondyloarthropathies, such as ankylosing spondylitis, and Sjogren's syndrome.

Pruritis treatable with a histamine H₄ receptor-modulating agent according to the invention includes that which is a symptom of allergic cutaneous diseases (such as atopic dermatitis and hives) and other metabolic disorders (such as chronic renal failure, hepatic cholestasis, and diabetes mellitus).

In a preferred embodiment, an active agent of the present invention is administered to treat allergy, asthma, autoimmune diseases, or pruritis.

Thus, the active agents may be used to treat subjects diagnosed with or suffering from a disease, disorder, or condition mediated through histamine H₄ receptor activity. The term "treat" or "treating" as used herein is intended to refer to administration of an active agent or composition of the invention to a subject for the purpose of effecting a therapeutic or prophylactic benefit through modulation of histamine H₄ receptor activity. Treating includes reversing, ameliorating, alleviating, inhibiting the progress of, lessening the severity of, or preventing a disease, disorder, or condition, or one or more symptoms of such disease, disorder or condition mediated through modulation of histamine H₄ receptor activity. The term "subject" refers to a mammalian patient in need of such treatment, such as a human. "Modulators" include both inhibitors and activators, where "inhibitors" refer to compounds that decrease, prevent, inactivate, desensitize or down-regulate histamine H₄ receptor expression or activity, and "activators" are compounds that increase, activate, facilitate, sensitize, or up-regulate histamine H₄ receptor expression or activity.

In treatment methods according to the invention, an effective amount of at least one active agent according to the invention is administered to a subject suffering from or diagnosed as having such a disease, disorder, or condition. An "effective amount" means an amount or dose sufficient to generally bring about the desired therapeutic or prophylactic benefit in patients in need of such treatment for the designated disease, disorder, or condition. When referring to modulating the target receptor, an "effective amount" means an amount sufficient to affect the activity of such receptor. Measuring the activity of the target receptor may be performed by routine analytical methods. Target receptor modulation is useful in a variety of settings, including assays. Effective amounts or doses of the active agents of the present invention may be ascertained by routine methods such as modeling, dose escalation studies or clinical trials, and by taking into consideration routine factors, e.g., the mode or route of administration or drug delivery, the pharmacokinetics of the agent, the severity and course of the disease, disorder, or condition, the subject's previous or ongoing therapy, the subject's health status and response to drugs, and the judgment of the treating physician. An exemplary dose is in the range of from about 0.001 to about 200 mg of active agent per kg of subject's body weight per day, preferably about 0.05 to 100 mg/kg/day, or about 1 to 35 mg/kg/day, or about 0.1 to 10 mg/kg daily in single or divided dosage units (e.g., BID, TID, QID). For a 70-kg human, an illustrative range for a suitable dosage amount is from about 0.05 to about 7 g/day, or about 0.2 to about 2.5 g/day.

Once improvement of the patient's disease, disorder, or condition has occurred, the dose may be adjusted for preventative or maintenance treatment. For example, the dosage or the frequency of administration, or both, may be reduced as a function of the symptoms, to a level at which the desired therapeutic or prophylactic effect is maintained. Of course, if symptoms have been alleviated to an appropriate level, treatment may cease. Patients may, however, require intermittent treatment on a long-term basis upon any recurrence of symptoms.

In addition, the active agents of the invention may be used in combination with additional active ingredients in the treatment of the above conditions. The additional active ingredients may be co-administered separately with an active agent of formula (I) or included with such an agent in a pharmaceutical composition according to the invention. In an exemplary embodiment, additional active ingredients are those that are known or discovered to be effective in the treatment of conditions, disorders, or diseases mediated by histamine H₄ receptor activity, such as another histamine H₄ receptor modulator or a compound active against another target associated with the particular condition, disorder, or disease. The combination may serve to increase efficacy (e.g., by including in the combination a compound potentiating the potency or effectiveness of an agent according to the invention), decrease one or more side effects, or decrease the required dose of the active agent according to the invention.

The active agents of the invention are used, alone or in combination with one or more additional active ingredients, to formulate pharmaceutical compositions of the invention. A pharmaceutical composition of the invention comprises an effective amount of at least one active agent in accordance with the invention. Such compositions may further comprise a pharmaceutically acceptable excipient.

A "pharmaceutically acceptable excipient" refers to a substance that is non-toxic, biologically tolerable, and otherwise biologically suitable for administration to a subject, such as an inert substance, added to a pharmacological composition or otherwise used as a vehicle, carrier, or diluent to facilitate administration of a agent and that is compatible therewith. Examples of excipients include calcium carbonate, calcium phosphate, various sugars and types of starch, cellulose derivatives, gelatin, vegetable oils, and polyethylene glycols.

Delivery forms of the pharmaceutical compositions containing one or more dosage units of the active agents may be prepared using suitable pharmaceutical excipients and compounding techniques known or that become available to those skilled in the art. The compositions may be administered in the inventive methods by a suitable route of delivery, e.g., oral, parenteral, rectal, topical, or ocular routes, or by inhalation.

The preparation may be in the form of tablets, capsules, sachets, dragees, powders, granules, lozenges, powders for reconstitution, liquid preparations, or suppositories. Preferably, the compositions are formulated for intravenous infusion, topical administration, or oral administration.

For oral administration, the active agents of the invention can be provided in the form of tablets or capsules, or as a solution, emulsion, or suspension. To prepare the oral compositions, the active agents may be formulated to yield a dosage of, e.g., from about 0.05 to about 50 mg/kg daily, or from about 0.05 to about 20 mg/kg daily, or from about 0.1 to about 10 mg/kg daily.

Oral tablets may include the active ingredient(s) mixed with compatible pharmaceutically acceptable excipients such as diluents, disintegrating agents, binding agents, lubricating agents, sweetening agents, flavoring agents, coloring agents and preservative agents. Suitable inert fillers include sodium and calcium carbonate, sodium and calcium phosphate, lactose, starch, sugar, glucose, methyl cellulose, magnesium stearate, mannitol, sorbitol, and the like. Exemplary liquid oral excipients include ethanol, glycerol, water, and the like. Starch, polyvinyl-pyrrolidone (PVP), sodium starch glycolate, microcrystalline cellulose, and alginic acid are exemplary disintegrating agents. Binding agents may include starch and gelatin. The lubricating agent, if present, may be magnesium stearate, stearic acid or talc. If desired, the tablets may be coated with a material such as glyceryl monostearate or glyceryl distearate to delay absorption in the gastrointestinal tract, or may be coated with an enteric coating.

Capsules for oral administration include hard and soft gelatin capsules. To prepare hard gelatin capsules, active ingredient(s) may be mixed with a solid, semi-solid, or liquid diluent. Soft gelatin capsules may be prepared by mixing the active ingredient with water, an oil such as peanut oil or olive oil, liquid paraffin, a mixture of mono and di-glycerides of short chain fatty acids, polyethylene glycol 400, or propylene glycol.

Liquids for oral administration may be in the form of suspensions, solutions, emulsions or syrups or may be lyophilised or presented as a dry product for reconstitution with water or other suitable vehicle before use. Such liquid compositions may optionally contain: pharmaceutically-acceptable excipients such as suspending agents (for example, sorbitol, methyl cellulose, sodium alginate, gelatin, hydroxyethylcellulose, carboxymethylcellulose, aluminum stearate gel and the like); non-aqueous vehicles, e.g., oil (for example, almond oil or fractionated coconut oil), propylene glycol, ethyl alcohol, or water; preservatives (for example, methyl or propyl p-hydroxybenzoate or sorbic acid); wetting agents such as lecithin; and, if desired, flavoring or coloring agents.

The active agents of this invention may also be administered by non-oral routes. For example, compositions may be formulated for rectal administration as a suppository. For parenteral use, including intravenous, intramuscular, intraperitoneal, or subcutaneous routes, the agents of the invention may be provided in sterile aqueous solutions or suspensions, buffered to an appropriate pH and isotonicity or in parenterally acceptable oil. Suitable aqueous vehicles include Ringer's solution and isotonic sodium chloride. Such forms may be presented in unit-dose form such as ampules or disposable injection devices, in multi-dose forms such as vials from which the appropriate dose may be withdrawn, or in a solid form or pre-concentrate that can be used to prepare an injectable formulation. Illustrative infusion doses range from about 1 to 1000 [mu]/kg/minute of agent admixed with a pharmaceutical carrier over a period ranging from several minutes to several days.

For topical administration, the agents may be mixed with a pharmaceutical carrier at a concentration of about 0.1% to about 10% of drug to vehicle. Another mode of administering the agents of the invention may utilize a patch formulation to affect transdermal delivery.

Active agents may alternatively be administered in methods of this invention by inhalation, via the nasal or oral routes, e.g., in a spray formulation also containing a suitable carrier.

The compounds of the invention can be prepared according to processes within the skill of the art and/or according to processes of this invention, such as those described in the schemes and examples that follow and by matrix or combinatorial methods.

Illustrative preparations are presented in the following Schemes. Other compounds of the invention may be made in the same general way, using modifications that will be apparent to those of ordinary skill in the art.

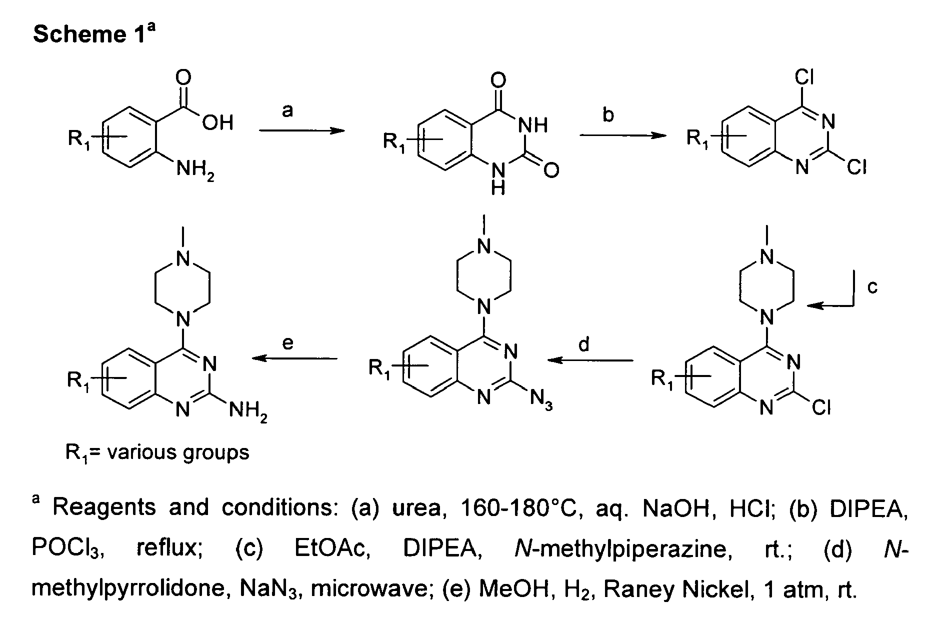

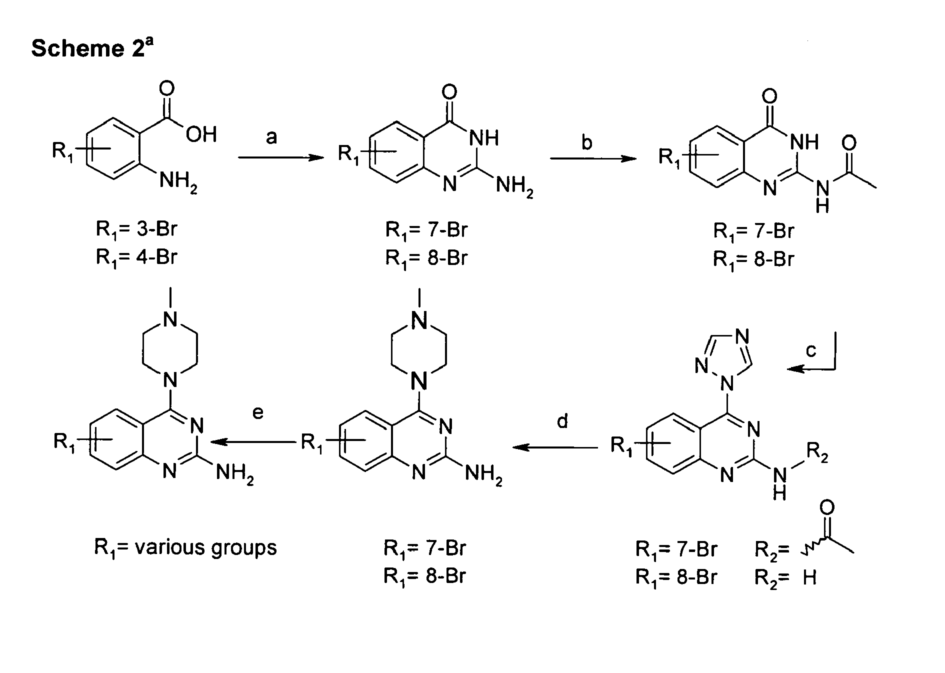

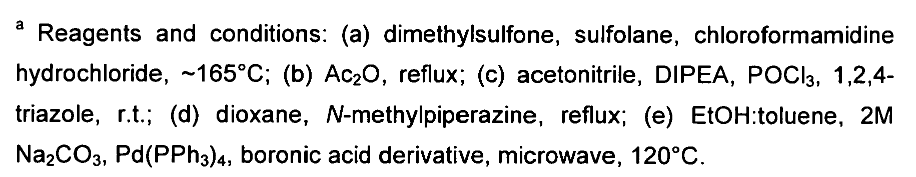

The following Examples illustrate the invention.

### General remarks

Chemicals and reagents were obtained from commercial suppliers and were used without further purification. Flash column chromatography was typically carried out on an Argonaut Flashmaster II flash chromatography system, using prepacked Silicycle Flash Si II columns with the UV detector operating at 254 nm. All ¹H-NMR spectra were measured on a Bruker 200, Bruker 250, Bruker 400 or Bruker 500. HRMS data was generated using a Bruker micrOTOF-Q (ESI).

### Chloroformamidine hydrochloride

100 mL (12 M) HCl was poured in a 250 mL flask and cooled to 0°C. Cyanamide (10 g) was added in portions and stirred for 2 hours at room temperature. The resulting mixture was then concentrated to about 40 mL and a white precipitate was formed. After cooling down to 0°C with an ice-bath, the precipitate was filtered off and the crystals were washed with HCl (6 M) to yield 6.80 gram as a solid.

### 2-amino-7-bromoquinazolin-4(3H)-one

A mixture of anthranilic acid (8.9 g), chloroformamidine hydrochloride (9.48 g), sulfolane (1.65 mL) and dimethylsulfone (19.8 g) was heated at 165°C for 1 hour. Then, water was added (300 ml) to the hot reaction mixture and the pH was adjusted to 7-8 by the addition of a aqueous ammonia solution in water (25%). The precipitate was collected by filtration and resuspended in MeOH (100 mL). Filtration and drying of he obtained solid in the presence of P₂O₅, yielded 9.9 g of the title compound as a solid. The crude product was used without further purification in the next step.

### 2-amino-8-bromoquinazolin-4(3H)-one

A mixture of anthranilic acid (5.0 g), chloroformamidine hydrochloride (5.32 g), sulfolane (0.92 mL) and dimethylsulfone (11.1 g) was heated at 165°C for 1 hour. Then, water was added (150 ml) and the pH was adjusted to 7-8 by the addition of an aqueous ammonia solution. The precipitate was collected by filtration and resuspended in H₂O:MeOH 1:1 (200 mL), filtered off and dried in the presence of P₂O₅, yielding 4.64 g of the crude title compound as a yellow solid. The crude product was used without further purification in the next step.

### N-(7-bromo-4-oxo-3,4-dihydroquinazolin-2-yl)acetamide

A suspension of 2-amino-7-bromoquinazolin-4(3*H*)-one (8.9 g) in acetic anhydride (80 mL) was heated at reflux for 1 hour. The resulting mixture was evaporated to dryness to yield 10.46 g of the crude title compound as a solid. The crude product was used without further purification in the next step.

### N-(8-bromo-4-oxo-3,4-dihydroquinazolin-2-yl)acetamide

A suspension of 2-amino-8-bromoquinazolin-4(3*H*)-one (4.64 g) in acetic anhydride (18 mL) was heated at reflux for 1 hour. The resulting mixture was evaporated to dryness to yield 5.45 g of the crude title compound as a solid. The crude product was used without further purification in the next step.

### General method A

### N-(7-bromo-4-(1H-1,2,4-triazol-1-yl)quinazolin-2-yl)acetamide

To a mixture of *N*-(7-bromo-4-oxo-3,4-dihydroquinazolin-2-yl)acetamide (10.44 g) and 1,2,4-triazole (25.57 g) in acetonitrile (370 mL) was added DIPEA (19.34 mL) and POCl₃ (17.03 mL). The resulting mixture was stirred for 16 hours at room temperature. The precipitate was filtered off and washed with both cold EtOH and diethylether to yield about 9.38 g of the crude title compound containing about 50% w/w of triazole by ¹H-NMR. The crude product was used without further purification in the next step.

### 8-bromo-4-(1H-1,2,4-triazol-1-yl)quinazolin-2-amine

Prepared according to general method A from *N*-(8-bromo-4-oxo-3,4-dihydroquinazolin-2-yl)acetamide (5.0 g), 1,2,4-triazole (12.25 g), POCl₃ (4.87 mL) and DIPEA (9.27 mL). Yield: 8.77 g of a solid containing about 62% w/w of triazole by ¹H-NMR. The crude product was used without further purification in the next step.

### 7-bromo-4-(4-methylpiperazin-1-yl)quinazolin-2-amine (VUF11272)

A mixture of N-(7-bromo-4-(1*H*-1,2,4-triazol-1-yl)quinazolin-2-yl)acetamide (5.0 g) and N-methylpiperazine (19.0 mL) in dioxane (200 mL) was refluxed for 45 minutes. The reaction mixture was concentrated under reduced pressure to yield a residue which was diluted with chloroform and 200 mL water

(1M NaOH). The aqueous layer was extracted with chloroform. The combined organic layers were dried over Na₂SO₄ and evaporated to dryness. The obtained solid was recrystallized from *iso*-propylalcohol to yield 2.12 g (6.58 mmol, 44%) of the title compound as a solid. ¹H-NMR (CDCl₃) δ (ppm) 7.64 (d, *J*= 2 Hz, 1H), 7.54 (d, *J*= 8.8 Hz, 1H), 7.18 (dd, *J*= 2.0 Hz, *J*= 8.8 Hz, 1H), 4.99 (bs, NH₂), 3.70 (t, *J*= 5.0 Hz, 4H), 2.57 (t, *J*= 5.0 Hz, 4H), 2.36 (s, 3H).

### 8-bromo-4-(4-methylpiperazin-1-yl)quinazolin-2-amine (VUF11271)

A mixture of 8-bromo-4-(1H-1,2,4-triazol-1-yl)quinazolin-2-amine (3.30) and N-methylpiperazine (14.3 mL) in dioxane (150 mL) was refluxed for 45 minutes. The reaction mixture was concentrated under reduced pressure to yield a residue, which was diluted with chloroform and 200 mL water (1M NaOH). The aqueous layer was extracted with chloroform. The combined organic layers were dried over Na₂SO₄ and evaporated to dryness. The solid was recrystallized from *iso*-propylalcohol to yield 1.79 g of the title compound as crystals. ¹H-NMR (CDCl₃) δ (ppm) 7.84 (dd, *J*= 1.3 Hz and *J*= 7.6 Hz, 1H), 7.67 (dd, *J*= 8.2 Hz and *J*= 1.3 Hz, 1H), 6.96-6.92 (m, 1H), 5.14 (bs, NH₂), 3.69 (t, *J*= 4.9 Hz, 4H), 2.58 (*J*= 4.9 Hz, 4H), 2.36 (s, 3H).

### General method B:

### 4-(4-methylpiperazin-1-yl)-7-phenylquinazolin-2-amine (VUF11261)

7-bromo-4-(4-methylpiperazin-1-yl)quinazolin-2-amine (200 mg) and phenylboronic acid (151.2 mg) were dissolved in 3 mL (1:1 EtOH:Toluene) and 2M Na₂CO₃ (342 µL) was added. The reaction mixture was flushed with N₂-gas for 15 minutes. Then, Pd(PPh₃)₄ (35.86 mg) was added and the resulting mixture was flushed for another 15 minutes with N₂-gas. Subsequently, the reaction mixture was typically heated for 1 hour at 120°C under microwave irradiation. The reaction mixture was diluted with chloroform and water (200 mL, 1 M NaOH) and the aqueous layer was extracted 3 times with -100 mL chloroform. The combined organic layers were dried over Na₂SO₄ and evaporation of the solvent gave the crude product that was typically purified by flash chromatography (SiO₂₎ by elution with EtOAc:MeOH:TEA 90:5:5 to yield 143 mg (0.45 mmol, 72%) of the title compound as a solid. ¹H-NMR (CDCl₃) δ (ppm) 7.77 (d, *J*= 8.6 Hz, 1H), 7.72-7.67 (m, 3H), 7.50-7.35 (m, 4H), 5.05 (bs, NH₂), 3.75 (t, *J*= 4.9 Hz, 4H), 2.60 (t, *J*= 4.9 Hz, 4H), 2.37 (s, 3H).

### 7-(2,6-dimethylphenyl)-4-(4-methylpiperazin-1-yl)quinazolin-2-amine (VUF11100)

Prepared according to general method B from 7-bromo-4-(4-methylpiperazin-1-yl)quinazolin-2-amine (200 mg) and 2,6-dimethylphenylboronic acid (186 mg, 1.24 mmol). Yield: 170 mg. ¹H-NMR (CDCl₃) δ (ppm) 7.78 (d, *J*= 8.4 Hz, 1H), 7.28 (d, *J*= 1.5 Hz, 1H), 7.21-7.09 (m, 3H), 6.92 (dd, *J*= 1.5 Hz, *J*= 8.4 Hz, 1H), 5.02 (bs, NH₂), 3.76 (t, *J*= 4.9 Hz, 4H), 2.36 (t, *J*= 4.9 Hz, 4H), 2.39 (s, 3H), 2.06 (s, 6H).

### 4-(2-amino-4-(4-methylpiperazin-1-yl)quinazolin-7-yl)benzonitrile (VUF11263)

Prepared according to general method B from 7-bromo-4-(4-methylpiperazin-1-yl)quinazolin-2-amine (200 mg) and 4-cyanophenylboronic acid (182.2 mg). Yield: 121 mg. ¹H-NMR (CDCl₃) δ (ppm) 7.80 (d, *J*= 8.6 Hz, 1H), 7.74 (app s, 4H), 7.68 (d, *J*= 1.7 Hz, 1H), 7.31 (dd, *J*= 1.7 Hz, *J*= 8.6 Hz, 1H), 5.08 (bs, NH₂) 3.75 (t, *J*= 4.8 Hz, 4H), 2.60 (t, *J*= 4.8 Hz, 4H), 2.37 (s, 3H); ¹³C-NMR (CDCl₃) δ (ppm) 166.15, 159.88, 154.45, 144.78, 143.06, 132.83, 128.01, 126.38, 124.18, 120.03, 11.87, 112.48, 111.82, 55.07, 49.74, 46.28.

### 7-(4-methoxyphenyl)-4-(4-methylpiperazin-1-yl)quinazolin-2-amine (VUF11262)

Prepared according to general method B from 7-bromo-4-(4-methylpiperazin-1-yl)quinazolin-2-amine (200 mg) and 4-methoxyphenylboronic acid (188.4 mg). Yield: 200 mg. ¹H-NMR (CDCl₃) δ (ppm) 7.72 (d, *J*= 8.6 Hz, 1H), 7.65 (d, *J*= 1.6 Hz, 1H), 7.61 (d, *J*= 8.7 Hz, 2H), 7.31 (dd, *J*= 1.6 Hz, *J*= 8.6Hz, 1H), 6.97 (d, *J*= 8.7 Hz, 2H), 5.17 (bs, NH₂), 3.83 (s, 3H), 3.72 (t, *J*= 4.7 Hz, 4H), 2.57 (t, *J*= 4.7 Hz, 4H), 2.35 (s, 3H).

### 7-(furan-3-yl)-4-(4-methylpiperazin-1-yl)quinazolin-2-amine (VUF11264)

Prepared according to general method B from 7-bromo-4-(4-methylpiperazin-1-yl)quinazolin-2-amine (200 mg) and furan-3-ylboronic acid (138.7 mg). ¹H-NMR (CDCl₃) δ (ppm) 7.83 (s, 1H), 7.69 (d, *J*= 8.6 Hz, 1H), 7.58 (d, *J*= 1.7 Hz, 1H), 7.49 (t, *J*= 1.6 Hz, 1H), 7.26-7.22 (m, 1H), 6.77 (d, *J*= 1.0 Hz, 1H), 5.03 (bs, NH₂), 3.72 (t, *J*= 4.9 Hz, 4H), 2.59 (t, *J*= 4.9 Hz, 4H), 2.36 (s, 3H).

### 4-(4-methylpiperazin-1-yl)-8-phenylquinazolin-2-amine (VUF11099)

Prepared according to general method B from 8-bromo-4-(4-methylpiperazin-1-yl)quinazolin-2-amine (200 mg) and phenylboronic acid (151.2 mg). Yield: 151 mg. ¹H-NMR (CDCl₃) δ (ppm) 7.73 (dd, *J*= 1.4 Hz and *J*= 8.3 Hz, 1H), 7.66 (d, *J*= 7.2 Hz, 2H), 7.58 (dd, *J*= 1.4 Hz, *J* = 7.2 Hz, 1H), 7.46-7.43 (m, 2H), 7.38-7.35 (m, 1H), 7.19-7.16 (m, 1H), 3.72 (app s, 4H), 2.63 (t, *J*= 4.8 Hz, 4H), 2.39 (s, 3H).

### 8-(2,6-dimethylphenyl)-4-(4-methylpiperazin-1-yl)quinazolin-2-amine (VUF11269)

Prepared according to general method B from 8-bromo-4-(4-methylpiperazin-1-yl)quinazolin-2-amine (200 mg) and 2,6-dimethylphenylboronic acid (186 mg). Yield: 63 mg. ¹H-NMR (CDCl₃) δ (ppm) 7.79 (dd, *J*= 1.5 Hz, *J*= 8.2 Hz, 1H), 7.34-7.29 (m, 2H), 7.20-7.15 (m, 3H), 5.10 (bs, NH₂), 3.76 (t, *J*= 4.8 Hz, 4H), 2.70 (t, *J*= 4.8 Hz, 4H), 2.46 (s, 3H), 2.01 (s, 6H).

### 4-(2-amino-4-(4-methylpiperazin-1-yl)quinazolin-8-yl)benzonitrile (VUF11268)

Prepared according to general method B from 8-bromo-4-(4-methylpiperazin-1-yl)quinazolin-2-amine (200 mg) and 4-cyanophenylboronic acid (182.2 mg). Yield: 122 mg. ¹H-NMR (CDCl₃) δ (ppm) 7.81-7.69 (m, 5H), 7.57 (dd, *J*= 1.4 Hz, *J*= 7.2 Hz, 1H), 7.18-7.15 (m, 1H), 3.74 (t, *J*= 4.9 Hz, 4H), 2.64 (t, *J*= 4.9 Hz, 4H), 2.40 (s, 3H).

### 8-(4-methoxyphenyl)-4-(4-methylpiperazin-1-yl)quinazolin-2-amine (VUF11098)

Prepared according to general method B from 8-bromo-4-(4-methylpiperazin-1-yl)quinazolin-2-amine (200 mg) and 4-methoxyphenylboronic acid (188.4 mg). Yield: 152 mg. ¹H-NMR (CDCl₃) δ (ppm) 7.69 (dd, *J*= 1.5 Hz, *J*= 8.3 Hz, 1H), 7.64-7.60 (m, 2H), 7.55 (dd, *J*= 1.5 Hz, *J*= 7.2 Hz, 1H), 7.17-7.10 (m, 1H), 6.99-6.96 (m, 2H), 4.91 (bs, NH₂), 3.85 (s, 3H), 3.69 (t, *J*= 4.9 Hz, 4H), 2.62 (t, *J*= 4.9 Hz, 4H), 2.38 (s, 3H).

### 8-(furan-3-yl)-4-(4-methylpiperazin-1-yl)quinazolin-2-amine (VUF11270)

Prepared according to general method B from 8-bromo-4-(4-methylpiperazin-1-yl)quinazolin-2-amine (200 mg) and furan-3-ylboronic acid (138.7 mg). Yield: 83 mg. ¹H-NMR (CDCl₃) δ (ppm) 8.57 (s, 1H), 7.71 (d, *J*= 7.2 Hz, 1H), 7.63 (d, *J*= 8.2 Hz, 1H) 7.48 (s, 1H), 7.14-7.08 (m, 1H), 6.90 (d, *J*= 0.9 Hz, 1H), 4.97 (bs, NH₂), 3.68 (t, *J*= 4.8 Hz, 4H), 2.60 (t, *J*= 4.8 Hz, 4H), 2.37 (s, 3H).

### 7-(furan-2-yl)-4-(4-methylpiperazin-1-yl)quinazolin-2-amine (VUF11295)

Prepared according to general method B from 7-bromo-4-(4-methylpiperazin-1-yl)quinazolin-2-amine (200 mg) and furan-2-ylboronic acid (138.7 mg). Yield: 169 mg. ¹H-NMR (CDCl₃) δ (ppm) 7.76 (d, *J*= 1.5 Hz, 1H), 7.72 (d, *J*= 8.7 Hz, 1H), 7.53 (d, *J*= 1.5 Hz, 1H), 7.43 (dd, *J*= 1.5 Hz, *J*= 8.7 Hz, 1H), 6.80 (d, *J*= 3.4 Hz, 1H), 6.52-6.50 (m, 1H), 4.89 (bs, NH₂), 3.72 (t, *J*= 4.9 Hz, 4H), 2.60 (t, *J*= 4.9 Hz, 4H), 2.38 (s, 3H)

### 4-(4-methylpiperazin-1-yl)-7-(thiophen-3-yl)quinazolin-2-amine (VUF11294)

Prepared according to general method B from 7-bromo-4-(4-methylpiperazin-1-yl)quinazolin-2-amine (200 mg) and 4,4,5,5-tetramethyl-2-(thiophen-3-yl)-1,3,2-dioxaborolane (243.1 mg). Yield: 174 mg. ¹H-NMR (CDCl₃) δ (ppm) 7.75-7.71 (m, 2H), 7.61-7.60 (m, 1H), 7.49 (d, *J*= 4.3 Hz, 1H), 7.43-7.36 (m, 2H), 4.91 (bs, NH₂), 3.73 (t, *J*= 4.8 Hz, 4H), 2.60 (t, *J*= 4.8 Hz, 4H), 2.38 (s, 3H)

HRMS data for selected compounds

| **Entry No.** | Compound | MF | MW Calc. | MW Found |
|---|---|---|---|---|
| 1 | VUF11272 | C13H17BrN5 | 322.0662 | 322.0655 |
| 2 | VUF11261 | C19H22N5 | 320.1870 | 320.1868 |
| 3 | VUF11100 | C21H26N5 | 348.2183 | 348.2169 |
| 4 | VUF11263 | C20H21N6 | 345.1822 | 345.1819 |
| 5 | VUF11262 | C20H24N5O | 350.1975 | 350.1965 |
| 6 | VUF11264 | C17H20N5O | 310.1662 | 310.1661 |
| 7 | VUF11271 | C13H17BrN5 | 322.0662 | 322.0657 |
| 8 | VUF11099 | C19H22H5 | 320.1870 | 321.1860 |
| 9 | VUF11269 | C21H26N5 | 348.2169 | 348.2170 |
| 10 | VUF11268 | C20H21 N6 | 345.1822 | 345.1813 |
| 11 | VUF11098 | C20H24N5O | 350.1975 | 350.1965 |
| 12 | VUF11270 | C17H20N5O | 310.1662 | 310.1666 |

## Claims

1. A compound of formula (I) 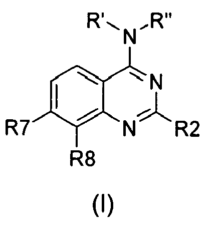 Wherein
R2 is H or NRxRy, where Rx and Ry are each independently selected from H, C₁₋₆ alkyl and (CH₂)₁₋₃C₃₋₇cycloalkyl; or Rx and Ry, together with the nitrogen atom to which they are bound form a 4, 5 or 6 membered heterocyclic group;
NR'R" is one of the following moieties, wherein R' and R" are taken together or separately as defined by each of one said moieties: 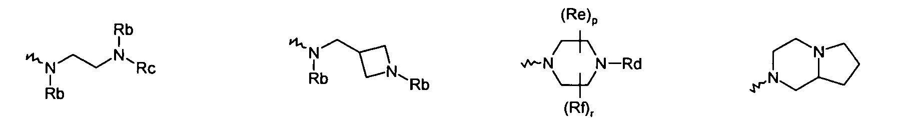 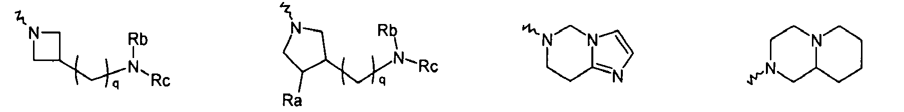 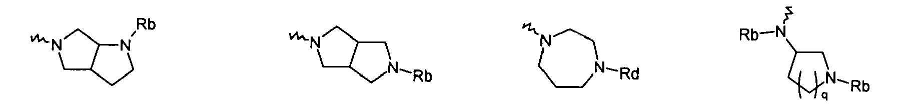 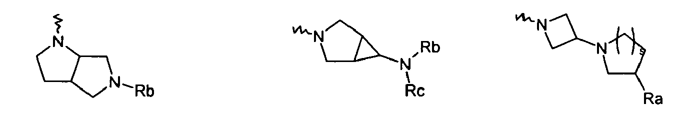 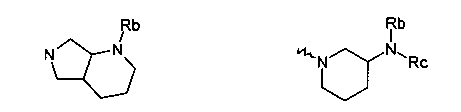 q is 0 or 1;
p is 0 or 1;
r is 0 or 1;
s is 0 or 1 or 2;
Ra is H or OH or F;
Rb and Rc are each independently H or C₁₋₃ alkyl;
Rd is H or OH or NH₂;
Re and Rf are each methyl, or Re and Rf taken together form a methylene or ethylene bridge; and
R₇ or R₈ are independently selected from H, F, Cl, Br, I, C₁₋₄ alkyl, C₂₋₅ alkenyl, C₂₋₅ alkynyl, C₁₋₄ alkoxy, C₁₋₄ alkylthio, (CH₂)₀₋₃C₃₋₇cycloalkyl, O-C₃₋₆ cycloalkyl, phenyl, benzyl, O-phenyl, NH-phenyl, S-phenyl, O-C₁₋₄ alkyl-phenyl, C₁₋₄ alkyl-phenyl, CF₃, O-CF₃, S-CF₃, hydroxy, nitro, cyano, O-C₁₋₄ alkyl-N(CH₃)₂, and NRmRn, wherein Rm and Rn are independently selected from H, C₁₋₄ alkyl, phenyl, benzyl and phenethyl, and wherein any phenyl, alkyl or cycloalkyl moiety is optionally substituted with 1 to 3 substituents selected from C₁₋₃ alkyl, halogen, hydroxy, amino and C₁₋₃ alkoxy;
or a pharmaceutically acceptable salt, ester, or solvate thereof.

2. A compound according to claim 1, wherein R2 is NH₂.

3. A compound according to claim 1 or claim 2, wherein NR'R"
is

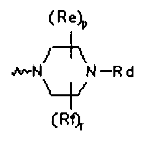

4. A compound according to claim 3, wherein Rd is methyl.

5. A compound according to claim 4, wherein Re and Rf form and ethylene bridge.

6. A compound according to any preceding claim, which is selected from:
7-methyl-4-(4-methylpiperazin-1-yl)quinazolin-2-amine;
8-methyl-4-(4-methylpiperazin-1-yl)quinazolin-2-amine;
7-bromo-4-(4-methylpiperazin-1-yl)quinazolin-2-amine;
8-bromo-4-(4-methylpiperazin-1-yl)quinazolin-2-amine;
4-(4-methylpiperazin-1-yl)-7-phenylquinazolin-2-amine;
7-(2,6-dimethylphenyl)-4-(4-methylpiperazin-1-yl)quinazolin-2-amine;
4-(2-amino-4-(4-methylpiperazin-1-yl)quinazolin-7-yl)benzonitrile;
7-(4-methoxyphenyl)-4-(4-methylpiperazin-1-yl)quinazolin-2-amine;
7-(furan-3-yl)-4-(4-methylpiperazin-1-yl)quinazolin-2-amine;
4-(4-methylpiperazin-1-yl)-8-phenylquinazolin-2-amine;
8-(2,6-dimethylphenyl)-4-(4-methylpiperazin-1-yl)quinazolin-2-amine;
4-(2-amino-4-(4-methylpiperazin-1-yl)quinazolin-8-yl)benzonitrile;
8-(4-methoxyphenyl)-4-(4-methylpiperazin-1-yl)quinazolin-2-amine;
8-(furan-3-yl)-4-(4-methylpiperazin-1-yl)quinazolin-2-amine;
7-(furan-2-yl)-4-(4-methylpiperazin-1-yl)quinazolin-2-amine and
4-(4-methylpiperazin-1-yl)-7-(thiophen-3-yl)quinazolin-2-amine.

7. A compound according to claim 6, which is selected from:
7-methyl-4-(4-methylpiperazin-1-yl)quinazolin-2-amine;
4-(4-methylpiperazin-1-yl)-7-phenylquinazolin-2-amine;
4-(2-amino-4-(4-methylpiperazin-1-yl)quinazolin-7-yl)benzonitrile;
7-(furan-3-yl)-4-(4-methylpiperazin-1-yl)quinazolin-2-amine;
7-(furan-2-yl)-4-(4-methylpiperazin-1-yl)quinazolin-2-amine and
4-(4-methylpiperazin-1-yl)-7-(thiophen-3-yl)quinazolin-2-amine.

8. A compound according to any preceding claim, for use in therapy of inflammatory disorders, allergic disorders, dermatological disorders, autoimmune disease, lymphatic disorders, and immunodeficiency disorders.

9. A compound according to claim 8, wherein the inflammatory disorder is selected from allergy, asthma, dry eye, chronic obstructed pulmonary disease (COPD), atherosclerosis, rheumatoid arthritis, multiple sclerosis, inflammatory bowel diseases (including colitis, Crohn's disease, and ulcerative colitis), psoriasis, pruritis, itchy skin, atopic dermatitis, urticaria (hives), ocular inflammation (e.g., post-surgical ocular inflammation), conjunctivitis, dry eye, nasal polyps, allergic rhinitis, nasal itch, scleroderma, autoimmune thyroid diseases, immune-mediated (also known as type 1) diabetes mellitus and lupus, which are **characterised by** excessive or prolonged inflammation at some stage of the disease. Other autoimmune diseases that lead to inflammation include Myasthenia gravis, autoimmune neuropathies, such as Guillain-Barré, autoimmune uveitis, autoimmune hemolytic anemia, pernicious anemia, autoimmune thrombocytopenia, temporal arteritis, anti-phospholipid syndrome, vasculitides, such as Wegener's granulomatosis, Behcet's disease, dermatitis herpetiformis, pemphigus vulgaris, vitiligio, primary biliary cirrhosis, autoimmune hepatitis, autoimmune oophoritis and orchitis, autoimmune disease of the adrenal gland, polymyositis, dermatomyositis, spondyloarthropathies, such as ankylosing spondylitis, and Sjogren's syndrome.
